# EUROPEAN PATENT SPECIFICATION

(11) **EP 3 153 133 B1**
(45) Date of publication and mention of the grant of the patent: **23.11.2022**
(21) Application number: 15803686.3
(22) Date of filing: 02.06.2015
(51) Int. Cl.: A61D 7/00, A61M 5/20, A61M 5/30, A61M 39/22

(54) **INJECTION SYSTEM USING NEEDLELESS SYRINGE**
EINSPRITZSYSTEM UNTER VERWENDUNG EINER NADELLOSEN SPRITZE
SYSTÈME D'INJECTION UTILISANT UNE SERINGUE SANS AIGUILLE

(30) Priority: 03.06.2014 JP 2014115109
(43) Date of publication of application: 12.04.2017
(73) Proprietor: Daicel Corporation, Osaka-shi, Osaka 530-0011 (JP)
(72) Inventor: YAMADA, Ryohei, Tatsuno-shi Hyogo 671-1681 (JP)
(74) Representative: McWilliams, David John
(86) International application number: PCT/JP2015/065833
(87) International publication number: WO 2015/186680

(56) References cited:
- WO-A1-89/04601
- WO-A1-94/17657
- WO-A1-2011/115496
- WO-A1-2012/135938
- JP-A- H03 502 408
- JP-A- H05 252 846
- JP-A- H07 308 140
- JP-A- H09 215 463
- JP-A- 2013 518 583
- US-B1- 6 436 709

## Description

### [TECHNICAL FIELD]

The present invention relates to a system for performing injection into an injection target by utilizing a needleless syringe for injecting an injection objective substance without using any injection needle.

### [BACKGROUND ART]

It is necessary to inoculate an vaccine in order to protect domestic animals from an infectious disease in a farm or the like. However, in general, the number of domestic animals is extremely large. Therefore, if the injection is performed for the domestic animals one by one in order to inoculate the vaccine, then an extremely long period of time is required as well, and the operation load exerted on an operator is not slight. In view of the above, a technique is disclosed in Patent Literature 1, which makes it possible to continuously inoculate the vaccine for the domestic animals. In this technique, a syringe is constructed as follows. That is, the flow of the compressed air is adjusted by an operator in a syringe body of the syringe by depressing and releasing a switch unit provided for a main body of the syringe to repeat the emission of an injection solution from the syringe body to the outside and the charging of the injection solution into the syringe body. Note that in the case of the syringe concerning this technique, an injection needle is used to deliver the injection solution to the domestic animal.

### [PRIOR ART LITERATURES]

### [PATENT LITERATURES]

Patent Literature 1: Japanese Utility Model Application Laid-Open No. 7-24314 A further known example of a method for the vaccination of fish can be found in WO 89/04601 A1. The method includes providing a system wherein the fish are anaesthetized and transported via a conveyer to a vaccination chamber removed from water, where the fish are vaccinated using a needle syringe.

### [SUMMARY OF THE INVENTION]

### [PROBLEMS TO BE SOLVED BY THE INVENTION]

According to the conventional technique, the depression/release operation of the switch, which is performed by the operator, is used as a trigger to repeat the charging and the emission of the injection solution with respect to the syringe having the needle. Therefore, it is necessary for the operator to perform the operation of the switch as described above after holding the syringe at the ready against the domestic animal or the like as the injection target (injection object) and piercing the injection target with the injection needle. Therefore, when the operator performs the injection into the domestic animal by himself/herself, it is necessary to operate the syringe in a state in which the movement of the domestic animal is suppressed. The load exerted on the operator is not necessarily light. Further, in order to mitigate the operation load exerted on one operator, it is necessary to allot a plurality of operators to the injection into the domestic animal.

Further, the syringe, which is used in the conventional technique, is the syringe having the needle. Therefore, the same injection needle is used for a plurality of domestic animals in some cases. In such a situation, it is impossible to eliminate such a possibility that any infectious disease may be spread among the domestic animals. Further, if the operator is brought in contact with the injection needle, a possibility also arises such that the operator may be infected.
Furthermore, in general, when the injection is performed for the domestic animals or the like, the number of the injection targets is large. Therefore, the load exerted on the operator is not slight as well in view of the injection management in which, for example, any marking is applied to the domestic animal having been subjected to the injection. In particular, when the injection is performed for a fish, the fish is returned to a predetermined place such as a stew (fishpond) or the like again after the termination of the injection. Therefore, the injection management is more difficult as compared with the domestic animal living on land.

In view of the above, taking the foregoing problems into consideration, an object of the present invention is to provide an injection system which mitigates the load exerted on an operator as far as possible and which suppresses the occurrence of various problems concerning the hygiene when the injection is performed for a large number of injection targets such as domestic animals or the like.

### [MEANS FOR SOLVING THE PROBLEMS]

In order to achieve the object as described above, the present invention adopts the following construction. Specifically, the present invention resides in an injection system using a needleless syringe which injects an injection objective substance into an injection target which is a living body capable of existing in water by emitting the injection objective substance from an emission port without using any injection needle; the system comprising a transfer passage which is arranged in water which connects a first space which is arranged in water for accommodating a plurality of the injection targets and a second space, which is arranged in water, as a transfer destination of the plurality of injection targets, the transfer passage being installed with the needleless syringe so that the emission port of the needleless syringe is open at inside of the transfer passage; a detecting unit which is capable of detecting transfer through the transfer passage in order for the injection target existing in the first space to go toward the second space; and a control unit which performs emission of the injection objective substance from the emission port of the needleless syringe for every injection target in accordance with the transfer of the injection target, if the transfer of the injection target is detected by the detecting unit; and a holding unit which has a holding plate which extends along the transfer passage, and which presses the holding plate against the injection target so that the injection target in the transfer passage is positioned with respect to the needleless syringe, and which temporarily stops the transfer of the injection target in the transfer passage to hold the injection target, and which allows the emission port of the needless syringe to abut against a body surface of the injection target on the basis of a detection result of the transfer of the injection obtained by the detecting unit.

In the case of the needleless syringe according to the present invention, the injection objective substance is not delivered to the inside of the injection target by using any injection needle. The injection is performed in such a manner that the injection objective substance is emitted from the main body of the syringe, the surface of the injection target is directly pierced by the injection objective substance by using the emission energy, and the injection objective substance is send into the injection target. Then, the injection objective substance, which is emitted by the needleless syringe, contains a component to be delivered to the injection target. Therefore, it is allowable that the physical form of the injection objective substance in the needleless syringe is any one of a fluid including, for example, a liquid and a gel form, a powder, and a granular solid or the like, provided that at least the emission can be performed from the main body of the syringe. For example, the injection objective substance may be a liquid. Alternatively, the injection objective substance may be a solid in a gel form provided that the fluidity, which enables the emission, is secured, even in the case of the solid. Then, the injection objective substance includes the component to be sent to the objective portion of the injection target. The component may exist in a state in which the component is dissolved in the injection objective substance. Alternatively, the component may be in a state in which the component is merely mixed without being dissolved.

In this context, in the case of the injection system described above, the needleless syringe is provided at the transfer passage which connects the first space and the second space. The first space is constructed as follows. That is, the plurality of injection targets are accommodated therein. Each of the injection targets receives the injection of the injection objective substance from the needleless syringe which is provided at the transfer passage during the process in which each of the injection targets in the first space is transferred to the second space via the transfer passage.

In this process, the transfer, which is performed for the injection target to go toward the second space via the transfer passage, is detected by the detecting unit. Then, the injection operation of the needleless syringe is controlled by the control unit in accordance with the detection result. That is, when the detecting unit detects the successive transfer of the injection target to the second space at the transfer passage, then the control unit allows the injection objective substance to be emitted from the needleless syringe to each of the injection targets positioned in front of the emission port, and thus the injection into the injection target is realized.

In the case of the injection system constructed as described above, the detection of the transfer of the injection target at the transfer passage, which is performed by the detecting unit, is used as the trigger to control the emission of the injection objective substance by the needleless syringe. Therefore, it is unnecessary for the operator to touch the injection target in principle, and it is also unnecessary for the operator to touch the needleless syringe itself. Therefore, it is considered that the operation load exerted on the operator is extremely decreased. Further, the syringe itself does not have any needle. Therefore, the possibility is suppressed to induce various problems concerning the hygiene (for example, spread of infectious disease as described above) resulting from the injection needle. Note that the detecting unit is provided to sense or predict the arrival of the injection target at the emission port of the needleless syringe, for which it is possible to use a sensor which applies, for example, the ultrasonic wave or the infrared light to the injection target or a detecting device which is based on the image processing by using a camera. However, it is possible to use any arbitrary sensor in conformity with the place of installation, provided that the sensing can be performed as described above.

In this context, in the construction as described above, the operator is not necessarily precluded from touching the injection target and/or the needleless syringe for any predetermined purpose. For example, in order to correctly emit the injection objective substance to the injection target, it is also allowable that the operator touches the needleless syringe and adjusts it. Further, in another example, in order to smoothly transfer the injection target via the transfer passage, it is also allowable that the operator performs an operation, for example, to induce the injection target. Even in the situations as described above, it is easy to understand that the operation load exerted on the operator concerning the injection is greatly mitigated. Note that in relation to the induction of the injection target, the injection system may further comprise an inducing device which transfers the injection target existing in the first space to the second space via the transfer passage. Accordingly, it is unnecessary for the operator himself/herself to perform the operation to induce the injection target.

In this context, as for the needleless syringe according to the present invention, various known energy generating modes can be adopted as the energy source for the emission, provided that the emission of the injection objective substance caused by the control unit can be performed with respect to the injection objective substance by using the detection result obtained by the detecting unit as the trigger. For example, it is possible to use an igniter powder which is ignited by an ignition device and a gas producing agent which produces a gas by means of combustion. When the combustion energy of a propellant or explosive is utilized as the emission energy, the igniter powder may be, for example, any one of propellants including a propellant containing zirconium and potassium perchlorate, a propellant containing titanium hydride and potassium perchlorate, a propellant containing titanium and potassium perchlorate, a propellant containing aluminum and potassium perchlorate, a propellant containing aluminum and bismuth oxide, a propellant containing aluminum and molybdenum oxide, a propellant containing aluminum and copper oxide, and a propellant containing aluminum and iron oxide, or a propellant composed of a combination of a plurality of propellants described above. The feature of the igniter powder as described above is as follows. That is, the combustion product thereof does not contain any gas component at the ordinary temperature even if the combustion product is a gas in a high temperature state. Therefore, the combustion product is immediately condensed after the ignition. As a result, when the syringe of the present invention is used for the injection into the living body, it is possible to efficiently perform the injection into a shallower portion of the injection target area of the living body. Further, when the generated energy of the gas producing agent is utilized as the emission energy, it is also possible to use, as the gas producing agent, a single base smokeless propellant and various gas producing agents used for a gas generator (gas producer) for the air bag and a gas generator (gas producer) for the seat belt pretensioner.

Further, as an embodiment of the driving unit other than the above, the energy of an elastic member such as a spring or the like may be utilized as the emission energy for the injection objective substance. For example, an electromagnetic valve, a solenoid actuator or the like, which is driven by applying the voltage from a power source circuit, is utilized to release a piston from a fastened state, the piston being fixed by an urging spring. Accordingly, the accumulated elastic energy of the urging spring can be utilized as the emission energy.

Further, as another method, it is also allowable that the energy of a pressurized gas is utilized directly or indirectly as the emission energy for the injection objective substance. Specifically, the needleless syringe described above may be constructed to include a syringe which serves as a space for accommodating a predetermined volume of the injection objective substance; an air cylinder which has a piston formed to be capable of performing reciprocating motion by supplying and discharging pressurized air and an air valve for driving the piston and which pressurizes the injection objective substance accommodated in the syringe by means of the reciprocating motion of the piston; and a nozzle which includes the emission port for emitting the injection objective substance pressurized by the piston toward the injection target. On this premise, the injection system further comprises a gas supply device which supplies the pressurized air for driving the piston in the air cylinder to the needleless syringe. The piston of the air cylinder may emit the injection objective substance by the aid of a plunger or the like.

In the case of the needleless syringe constructed as described above, the energy of the pressurized gas supplied from the gas supply device is utilized as the emission energy of the injection objective substance. That is, the supplied pressurized gas allows the piston possessed by the air cylinder to perform the reciprocating motion. The injection objective substance is accommodated and charged into the syringe, and the injection objective substance is discharged from the syringe to the nozzle in accordance with the reciprocating motion. Then, the injection objective substance, which is discharged to the nozzle, is emitted from the emission port of the nozzle toward the injection target. Note that the pressure of the pressurized gas supplied by the gas supply device serves as the emission energy source of the injection objective substance. Therefore, it is preferable that the pressure of the pressurized gas is adjusted so that the emission of the injection objective substance suitable for the injection target is performed.

In this context, in the injection system described above, the needleless syringe may include a first valve which allows the injection objective substance to be capable of flowing in only one direction from a vial toward the syringe in a communication passage formed between the syringe and the vial that accommodates the injection objective substance; and a second valve which allows the injection objective substance to be capable of flowing in only one direction from the syringe toward the nozzle in a discharge passage formed between the nozzle and the syringe. Then, the injection objective substance may be supplied into the syringe from the vial via the communication passage when the first valve is in an open state and the second valve is in a closed state when the piston is moved backwardly in the air cylinder to provide a negative pressure in the syringe. Further, in place of the arrangement as described above, it is also allowable that the accommodation and the charging of the injection objective substance into the syringe and the discharge of the injection objective substance from the syringe may be realized by electronically controlling the open/closed states of the first valve and the second valve respectively.

Further, the injection system described above may further comprise an obstructing unit which obstructs the injection target having transferred from the first space to the second space from returning toward the first space, the obstructing unit being provided in a predetermined passage range disposed on a side of the second space as compared with an installation portion of the emission port of the needleless syringe in the transfer passage. When the obstructing unit is provided as described above, the injection target, for which the injection has been performed by means of the needleless syringe, can be physically suppressed from returning to the first space via the transfer passage. This arrangement greatly contributes to mitigate the load of the injection management exerted on the operator. Note that as an example of the specified structure of the obstructing unit, the obstructing unit may be composed of a plurality of inclined plates which are inclined toward the side of the second space as compared with the installation portion of the emission port in the transfer passage and which protrude to an inner side of the transfer passage. Note that it is also allowable to obstruct the injection target from returning to the first space by means of any other mode.

### [ADVANTAGEOUS EFFECT OF THE INVENTION]

According to the injection system concerning the present invention, the load exerted on the operator can be mitigated as far as possible, and the occurrence of various problems concerning the hygiene can be suppressed, when the injection is performed for a large number of injection targets such as domestic animals or the like.

### [BRIEF DESCRIPTION OF THE DRAWINGS]

Fig. 1 schematically shows an injection system using a needleless syringe according to the present invention.
Fig. 2 shows a schematic arrangement of the needleless syringe used in the injection system shown in Fig. 1.
Fig. 3 shows a schematic arrangement of a holding device used in the injection system shown in Fig. 1.
Fig. 4 shows a flow chart of an injection process executed by the injection system shown in Fig. 1.

### [EMBODIMENTS FOR CARRYING OUT THE INVENTION]

An explanation will be made below about an injection system 1 according to an embodiment of the present invention with reference to the drawings. Note that the arrangement of the following embodiment is shown by way of example, and the present invention is not limited to the arrangement of the embodiment.

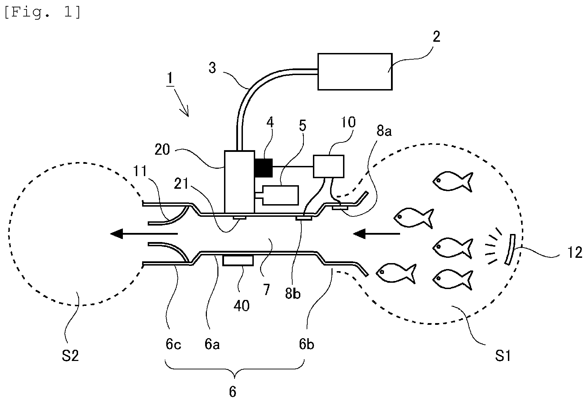

Fig. 1 shows a schematic arrangement of an injection system 1. The injection system 1 is the system for executing the injection of a drug solution or liquid medicine (corresponding to the injection objective substance of the present invention) by means of a needleless syringe 20 in order to prevent farmed fish in water from any infectious disease. Specifically, the injection of the drug solution is executed for the farmed fish during the process in which a plurality of the farmed fish existing in a first space S1 are transferred or moved to a second space S2 which is a space distinct from the first space S1. Note that in the following description of the present invention, the injection objective substance, which is injected into the injection target by means of the needleless syringe 20, is generally referred to as "drug solution". However, it is not intended thereby to limit the contents and the form of the substance to be injected. As for the injection objective substance, it is allowable that the component, which is to be delivered to the farmed fish or the like as the injection target, is either dissolved or not dissolved. Further, the specified form of the injection objective substance is also insignificant, for which it is possible to adopt various forms including, for example, liquid and gel form, provided that the injection objective substance can be emitted to the injection target by means of the needleless syringe 20.

In this case, the first space S1 and the second space S2 are the spaces which are formed while being isolated from the surroundings so that the farmed fish are transferable or movable via only a transfer space 7 formed by a transfer passage 6. Therefore, the farmed fish cannot come and go between the first space S1 and the second space S2 without passing through the transfer space 7. Further, the transfer space 7 approximately has such a cross-sectional area that only one farmed fish as the injection target of the drug solution can transfer or move therethrough. Therefore, when the farmed fish are transferred from the first space S1 to the second space S2, the farmed fish passes through the transfer space 7 one by one in front of ultrasonic sensors 8a, 8b and an emission port 21 as described later on. Note that a stew (fishpond) or the like, which is generally utilized to culture or breed fish, can be exemplified as the first space S1 and the second space S2 by way of example. In this case, the two stews are connected to one another by the transfer passage 6, and thus it is possible to form the injection system 1 shown in Fig. 1.

In this case, the transfer passage 6 is formed by a first space side connecting portion 6b which is connected to the first space S1, a second space side connecting portion 6c which is connected to the second space S2, and a main passage body 6a which connects the both connecting portions. Then, the farmed fish, which exists in the first space S1, can enter the transfer space 7 from the first space side connecting portion 6b, and the farmed fish can be transferred to the second space S2 via the transfer space 7 in the second space side connecting portion 6c and the main passage body 6a. Then, an entry obstructing device 11 (this device corresponds to the obstructing unit according to the present invention), which is formed by a plurality of inclined plates inclined toward the side of the second space S2 and arranged to protrude toward the transfer space 7 from the inner wall of the second space side connecting portion 6c, is provided for the second space side connecting portion 6c. The inclined plates of the entry obstructing device 11 are inclined toward the side of the second space S2. Therefore, the farmed fish can be transferred or moved from the first space S1 to the second space S2 relatively easily. On the other hand, the entry port into the transfer space 7 is formed to be relatively small, as viewed by the farmed fish having been transferred to the second space S2. Therefore, it is possible to obstruct the farmed fish from returning from the second space S2 to the first space S1.

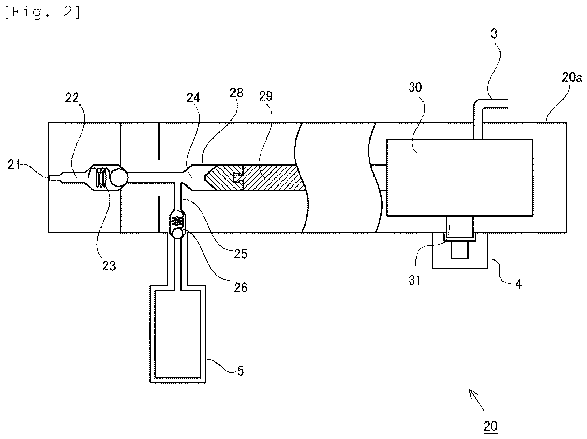

Further, the needleless syringe 20 is installed at the main passage body 6a. Note that when the needleless syringe 20 is installed at the main passage body 6a, a state is given, in which the emission port 21 is exposed to the transfer space 7. An exemplary structure of the needleless syringe 20 will now be explained on the basis of Fig. 2. Fig. 2 shows a sectional view illustrating the needleless syringe 20 as taken in the longitudinal direction thereof. The left side shown in Fig. 2 is the forward end side of the needleless syringe 20, on which the emission port 21 is arranged. Note that the description "forward end side" in this application refers to the side near to the emission port 21 as compared with the "proximal end side". Therefore, the left side as viewed in Fig. 2 corresponds to the "forward end side".

The needleless syringe 20 has an air cylinder including an air valve 30 which accumulates and releases the pressurized air supplied from the outside and a piston 29 which is formed to be capable of performing the reciprocating motion in a sliding hole 28 formed in a main syringe body 20a by utilizing the pressurized air. Specifically, the pressurized air is supplied to the air valve 30 via a supply tube 3 from a pressurized air supply apparatus (compressor) 2 arranged outside the syringe. Then, those provided in the air valve 30 are an accumulating chamber (not shown) which accumulates the supplied pressurized air and a releasing unit (not shown) which releases the accumulated pressurized air toward the sliding hole 28 in which the piston 29 is arranged. Note that the switching or changeover of the accumulation and the release of the pressurized air in the air valve 30 is controlled by the depression and the release of a changeover button 31. Then, the depression and the release of the changeover button 31 are executed by a solenoid startup device 4 which is driven in accordance with a startup signal fed from a control device 10 shown in Fig. 1 (this device corresponds to the control unit according to the present invention). When the solenoid startup device 4 receives the startup signal from the control device 10, then the driving current flows through a solenoid contained therein, and a plunger is driven by the magnetic force generated by the solenoid to make it possible to depress the changeover button 31. Then, the driving current is stopped, then the plunger returns, and the changeover button 31 is released from the depression.

In this arrangement, a positioning spring (not shown), which determines the relative position of the piston 29 with respect to the air valve 30, is provided between the air valve 30 and the piston 29 arranged in the sliding hole 28. Therefore, the piston 29 is movable in the sliding hole 28. However, the piston 29 is in a state in which the urging force is received from the positioning spring during the movement thereof. Note that the state shown in Fig. 2 represents the state in which the pressurized air is accumulated in the air valve 30, i.e., the state in which the pressurized air is not released from the air valve 30 with respect to the piston 29. The relative position of the piston 29 with respect to the air valve 30 in this state resides in the state in which the piston 29 is arranged on the side of the air valve 30 by means of the urging force of the positioning spring.

Further, a syringe 24, which is a space for accommodating the drug solution (injection solution or parenteral solution) to be emitted by the needleless syringe 20, is formed on the forward end side of the piston 29 (side opposite to the air valve 30) in the state shown in Fig. 2. A drug solution supply passage 25 (this passage corresponds to the communication passage according to the present invention) is open at a position at which no interference occurs with the piston 29 which makes the reciprocating motion in the syringe 24. The vial 5, which accumulates the drug solution, is connected via the first valve 26 to the drug solution supply passage 25. When the first valve 26 is in the open state, the drug solution can be supplied from the vial 5 via the drug solution supply passage 25 to the syringe 24. Note that the first valve 26 performs the regulation so that the drug solution flows in only one direction directed from the vial 5 to the syringe 24. Therefore, when the drug solution flows while being directed from the syringe 24 to the vial 5, the first valve 26 is closed by the force exerted by the flow. On this account, as for the first valve 26, the valve is pressed in the direction directed to the vial 5 by means of elastic means such a spring or the like, and the first valve 26 is normally closed. However, the opening/closing of the first valve 26 may be electronically controlled by the control device 10.

Further, the syringe 24 is communicated on the forward end side with a discharge passage 22 via the second valve 23. The end portion of the discharge passage 22, which is disposed on the forward end side, corresponds to the emission port 21 described above. Therefore, when the second valve 23 is in the open state, the drug solution contained in the syringe 24 can be emitted from the emission port 21 via the discharge passage 22. Note that the second valve 23 performs the regulation so that the drug solution flows in only one direction directed from the syringe 24 toward the discharge passage 22. The second valve 23 is normally closed while the valve is pressed in the direction directed to the syringe 24 by means of elastic means such as a spring or the like. Then, the second valve 23 is opened by the force exerted by the flow only when the drug solution flows from the syringe 24 toward the discharge passage 22. However, the opening/closing of the second valve 23 may be also electronically controlled by means of the control device 10.

In the needleless syringe 20 constructed as described above, the drug solution contained in the vial 5 can be continuously emitted in accordance with the reciprocating motion of the piston 29 in the sliding hole 28 and the opening/closing of the first valve 26 and the second valve 23. The operation for continuously emitting the drug solution will be explained below.

### (1) First operation

In the first operation, the first valve 26 is closed, the second valve 23 is closed, and the pressurized air is sent from the compressor 2 to the air valve 30. Then, the pressurized air is accumulated to arrive at a predetermined pressure in the valve 30. The predetermined pressure is the pressure at which the piston 29 can be pressurized so that the drug solution can be emitted when the pressurized air is released in accordance with the second operation as described later on. Note that during the first operation, such a state is given that the drug solution is charged into the syringe 24 as a result of the third operation described later on.

### (2) Second operation

In the second operation, the pressurized air, which is accumulated in the air valve 30, is released for the piston 29 in accordance with the depression of the changeover button 31. As a result, the piston 29 is propelled toward the forward end side in the sliding hole 28 against the urging force received from the positioning spring. Then, the syringe 24 is filled with the drug solution, and hence the drug solution is discharged to the outside of the syringe 24 by being propelled by the piston 29. In this situation, the drug solution flows toward the first valve 26 and the second valve 23. However, the first valve 26 is closed in accordance with the flow of the drug solution, and the drug solution does not flow into the vial 5. Further, the second valve is released in accordance with the flow of the drug solution. The drug solution flows toward the discharge passage 22, and the drug solution is emitted from the emission port 21.

### (3) Third operation

In the third operation performed after the emission of the drug solution in accordance with the second operation, the changeover button 31 is released from the depression. Accordingly, the pressurized air, which is released in the second operation, is released to the outside of the main syringe body 20a, and the piston 29 is restored to the original position (position shown in Fig. 2), i.e., the position of the piston in the first operation, by means of the positioning spring. Then, the volume of the syringe 24 is restored in accordance with the restoring operation of the piston 29, and the interior of the syringe 24 is in a negative pressure state. Therefore, the urging force is also applied from the elastic means, the second valve 23 is closed, and the first valve 26 is opened. The drug solution, which is accumulated in the vial 5, is sucked into the syringe 24, and the syringe 24 is filled therewith. Then, the first operation and the followings are repeated again after the termination of the third operation. Thus, it is possible to perform the continuous injection by using the needleless syringe 20.

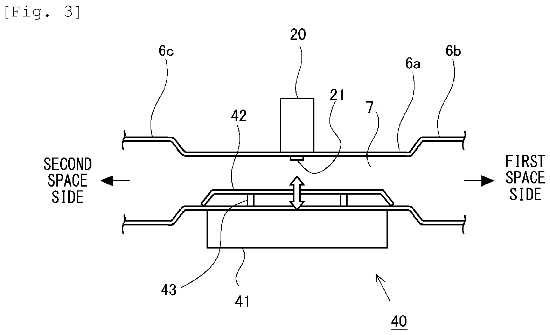

In this context, with reference to Fig. 1 again, the holding device 40 is arranged at the position of the main passage body 6a opposed to the emission port 21 of the needleless syringe 20 in the injection system 1. The holding device 40 is the device which holds the farmed fish to press the farmed fish against the emission port 21 so that the farmed fish is brought in contact therewith, when the farmed fish is transferred through the transfer space 7 from the first space S1 toward the second space S2. The schematic arrangement of the holding device 40 will be explained on the basis of Fig. 3. The holding device 40 has a main device body 41 which is arranged outside the main passage body 6a, a holding plate 42 which extends along the main passage body 6a, and feet 43. The holding plate 42 is connected with the main device body 41 by the aid of the feet 43, and the holding plate 42 is arranged in the transfer space 7 in the main passage body 6a. Then, the foot 43 is constructed so that the protruding amount from the main device body 41 can be adjusted. When the feet 43 protrude by larger amounts from the main device body 41, then the distance between the holding plate 42 and the emission port 21 is narrowed, and it is possible to hold the farmed fish transferred through the transfer space 7 and press the body thereof against the emission port 21. Note that the protruding amounts of the feet 43 are controlled by the control device 10. Further, a preferred sealing treatment is applied so that no water enters the interior of the main device body 41 when the protruding amounts of the feet 43 are changed. Note that the protrusion and the accommodation of the foot 43 may be performed in accordance with the supply and exclusion of the compressed air from the compressor 2.

In the injection system 1 shown in Fig. 1, the needleless syringe 20 and the holding device 40 are appropriately controlled by the control device 10, and thus the injection process with the drug solution is executed for the farmed fish which is transferred from the first space S1 to the second space S2. Then, in order to facilitate the transfer of the farmed fish to the second space S2, an inducing device 12 is installed in the first space S1. For example, the inducing device 12 may be constructed as follows. That is, the plurality of farmed fish existing in the first space are excited by applying the stimulation of light, sound or the like thereto. The farmed fish are expelled to the second space via the transfer space 7 that is also the sole space through which the farmed fish can escape from the first space S1. Note that the stimulation of light and/or sound to be applied can be appropriately determined taking the biological characteristics of the farmed fish as the target or object into consideration.

On the other hand, another method is also available for the inducing device 12. That is, it is also allowable to use such an inducing device that the volume of the first space S1 is gradually decreased, or the spatial shape of the first space S1 is deformed to form a state in which the farmed fish hardly stay in the first space S1 physically. Further, the following arrangement can be also adopted as the inducing device 12. That is, although the size and the spatial shape of the first space S1 itself are not changed, the physical contact is made with the farmed fish, and the farmed fish are expelled to the first space side connecting portion 6b.

Further, ultrasonic sensors 8a, 8b, which detect the presence of the farmed fish passing through the corresponding transfer space 7 by means of the ultrasonic wave, are installed respectively at the first space side connecting portion 6b and in the vicinity of the connecting portion of the main passage body 6a with respect to the first space side connecting portion 6b. The ultrasonic sensors 8a, 8b are installed while being separated from each other by an appropriate distance so that the respective detection ranges are not overlapped with each other. The ultrasonic sensors 8a, 8b are electrically connected to the control device 10 so that the detection signals of the respective ultrasonic sensors are delivered to the control device 10.

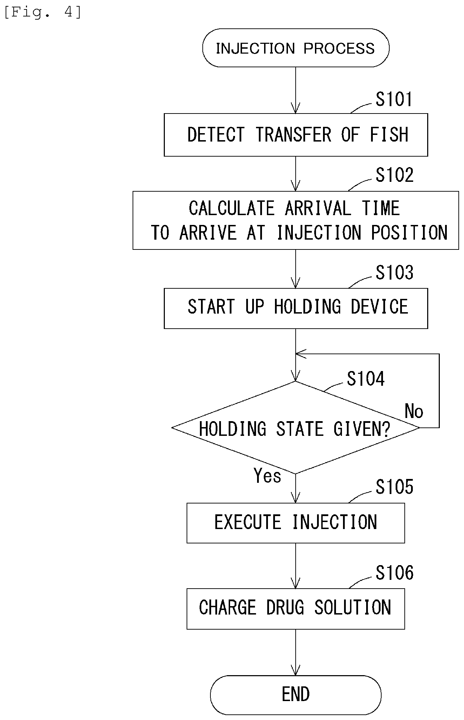

In the injection system 1 constructed as described above, the injection process shown in Fig. 4 is repeatedly executed by the control device 10. Accordingly, the continuous injection of the drug solution is realized for the farmed fish as described above. The control device 10 is a computer having a memory and a calculating device. The injection process shown in Fig. 4 is executed by executing a predetermined control program.

At first, in S101, the transfer in the transfer space 7 ranging from the first space side connecting portion 6b to the main passage body 6a is detected for the farmed fish existing in the first space S1 on the basis of the detection signals fed from the ultrasonic sensors 8a, 8b. Specifically, at first, if the presence of any object is detected by the ultrasonic sensor 8b within a predetermined time range from the point in time at which the presence of any object is detected by the ultrasonic sensor 8a disposed near to the first space S1, it is detected that "the farmed fish in the first space S1 transfers or moves to the second space via the transfer space 7". Note that the predetermined time range is the time width which is assumed to be required to pass between the two ultrasonic sensors if the farmed fish performs the assumed transfer or movement. Therefore, in this embodiment, if the detection result is obtained by the ultrasonic sensor 8b at a time interval deviated from the predetermined time range, or if the detection is performed by only the ultrasonic sensor 8a, then it is considered that the farmed fish does not transfer toward the second space S2 in the transfer space 7 in this state, and it is unnecessary to perform the injection with the needleless syringe 20. Note that when the transfer of the farmed fish is detected, it is also allowable to judge that the transfer of the farmed fish is detected if any condition other than the detection condition described above is established. Further, in order to perform the correct detection, a device, which detects the passage of the injection target through the transfer space 7 by means of a camera or the like in accordance with the image analysis, can be also used in combination with the ultrasonic sensor. If the process of S101 is terminated, the routine proceeds to S102.

In S102, the time, at which the objective farmed fish arrives at the injection position for the needleless syringe 20, is calculated on the basis of the detection result obtained in S101. Specifically, when the presence of the farmed fish is detected by the ultrasonic sensors 8a, 8b, the movement speed, which is provided when the farmed fish transfers or moves through the transfer space 7, is calculated from the interval between the respective detection times and the installation distance between the both ultrasonic sensors. Then, the time, which is required until the farmed fish passes in front of the emission port 21 of the needleless syringe 20 after the farmed fish passes in front of the ultrasonic sensor 8b, can be calculated as the arrival time on the basis of the movement speed and the installation position of the needleless syringe 20 (for example, the distance between the ultrasonic sensor 8b and the needleless syringe 20). If the process of S102 is terminated, the routine proceeds to S103.

In S103, the holding device 40 is started up so that the farmed fish is held by the holding plate 42 to bring the body thereof in contact with the emission port 21 at the point in time at which it is assumed that the farmed fish passes in front of the emission port 21 of the needleless syringe 20 on the basis of the arrival time calculated in S102.

In S104, the holding device 40 is started up to judge whether or not such a state is given that the farmed fish is held by the holding plate 42. For example, the holding state of the farmed fish can be also judged by detecting the force transmitted via the feet 43 by means of a force sensor (not shown) installed in the main device body 41. Further, in another method, the judgment of the holding state as described above may be replaced with the following procedure. That is, if the protruding amount of the foot 43 is previously determined while considering the assumed size of the farmed fish without utilizing the detecting device such as the force sensor or the like, it is judged whether or not the foot 43 protrudes by a predetermined protruding amount in accordance with the startup of the holding device 40. If the affirmative judgment is made in S104, the process proceeds to S105. If the negative judgment is made, the judgment of S104 is performed again.

Subsequently, in S105, the injection of the drug solution is executed by the needleless syringe 20 in the state in which the farmed fish as the injection target is held by the holding plate 42 of the holding device 40. The injection of the drug solution is realized in accordance with the first operation and the second operation described above. If the process of S105 is terminated, the routine proceeds to S106. Then, in S106, the charging of the drug solution into the syringe 24 is performed in accordance with the third operation described above after the injection of the drug solution by the needleless syringe 20 is completed in S105.

As described above, according to the foregoing injection process, when the farmed fish, which is the injection target existing in the first space S1, transfers to the second space S2 via the transfer space 7, then the transfer is detected by the ultrasonic sensors 8a, 8b, and the farmed fish is automatically positioned with respect to the needleless syringe 20. In this positioned state, the emission port 21 is brought in contact with the body of the farmed fish. Therefore, it is possible to preferably realize the injection with the needleless syringe 20 which emits the drug solution by utilizing the pressurized air. Further, the obstructing device 11, which has the inclined plate, is arranged at the second space side connecting portion 6c. Accordingly, it is possible to avoid such a state that the farmed fish, for which the injection has been performed and which has transferred to the second space S2, returns to the first space S1 and it is difficult to manage the injection. Therefore, it is possible to realize the continuous automatic injection of the drug solution with respect to the plurality of farmed fish by repeatedly executing the foregoing injection process by the control device 10. Note that a gate may be arranged at the first space side connecting portion 6b to obstruct the entry of the farmed fish so that the next injection target (farmed fish) does not enter the transfer passage 6 until the injection with the needleless syringe 20 is completed. The gate may be opened/closed by the control device 10. For example, the gate may be once closed after the passage is detected by the ultrasonic sensor 8a, and the gate may be opened again when the injection is completed (when the holding device 40 is released).

### <First modified embodiment>

In the injection system 1 shown in Figs. 1 to 3, the emission port 21 of the needleless syringe 20 and the holding plate 42 of the holding device 40 are arranged so that they are opposed to one another at the main passage body 6a. In the case of this arrangement, the body of the farmed fish is pressed against the emission port 21 by the holding plate 42. On the other hand, as a modified embodiment thereof, it is also allowable that the needleless syringe 20 and the holding device 40 are constructed integrally so that the emission port 21 of the needleless syringe 20 is open on the holding plate 42. In this case, the emission port 21 is brought in contact with the body of the farmed fish together with the holding plate 42, and then the farmed fish is pressed against the inner wall of the opposing main passage body 6a. Even in the case of the embodiment as described above, when the injection by the needleless syringe 20 is executed, such a state is given that the emission port 21 is brought in contact with the body of the farmed fish. Therefore, it is possible to realize the preferred emission of the drug solution.

### <Second modified embodiment>

In the case of the needleless syringe 20 used in the embodiment described above, the release energy based on the pressurized air is utilized for the propelling force of the piston 29. In place of this mode, it is also allowable that a syringe, which carries a plurality of igniters or exploders that carry a propellant and which realizes the continuous emission of the drug solution by successively starting up the plurality of igniters, is adopted as the needleless syringe 20. In this case, the igniter and the piston 29 are arranged so that the combustion product, which is produced by the combustion of the propellant in the igniter, pressurizes the piston 29. Further, a gas producing agent or the like, which is combusted by the combustion product and which produces the gas, can be further arranged between each of the igniters and the piston 29 as well. The gas producing agent is exemplified, for example, by a single base smokeless propellant (gunpowder) composed of 98% by mass of nitrocellulose, 0.8% by mass of diphenylamine, and 1.2% by mass of potassium sulfate. Further, it is also possible to use various gas producing agents used for a gas generator (gas producer) for the air bag and a gas generator (gas producer) for the seat belt pretensioner.

Note that the igniter powder, which is usable in the igniter, is preferably exemplified by a propellant containing zirconium and potassium perchlorate (ZPP), a propellant containing titanium hydride and potassium perchlorate (THPP), a propellant containing titanium and potassium perchlorate (TiPP), a propellant containing aluminum and potassium perchlorate (APP), a propellant containing aluminum and bismuth oxide (ABO), a propellant containing aluminum and molybdenum oxide (AMO), a propellant containing aluminum and copper oxide (ACO), a propellant containing aluminum and iron oxide (AFO), and propellants composed of combinations of a plurality of the propellants described above. The propellants as described above have the following characteristics. That is, the plasma at a high temperature and a high pressure is generated during the combustion immediately after the ignition. However, when the temperature becomes the ordinary temperature, and the combustion product is condensed, then the generated pressure is suddenly lowered, because no gas component is contained. It is also allowable that any propellant other than the above is used as the igniter powder, provided that the appropriate injection can be performed.

### <Third modified embodiment>

The injection system 1 concerning the embodiment described above realizes the injection of the drug solution with respect to the farmed fish existing in water. However, in place of this mode, it is also possible to make the application to any domestic animal (for example, cattle and pig) existing on land. In this case, no water exists between the needleless syringe 20 and the domestic animal. Therefore, on condition that the emission speed of the drug solution emitted from the needleless syringe 20 is sufficiently high, the drug solution can be injected without causing any trouble in some cases, even when the emission port 21 and the body of the domestic animal are somewhat separated from each other. In such a situation, it is not necessarily indispensable to provide the holding device 40 shown in Figs. 1 and 3. Of course, it is also allowable to install the holding device 40 for the injection system 1 in order to perform the injection of the drug solution more stably.

### [DESCRIPTION OF THE REFERENCE SIGNS]

1: injection system, 2: pressurized air supply apparatus (compressor), 4: solenoid startup device, 5: vial, 6: transfer passage, 7: transfer space, 8a, 8b: ultrasonic sensor, 10: control device, 11: obstructing device, 12: inducing device, 20: needleless syringe, 21: emission port, 22: discharge passage, 23: second valve, 24: syringe, 25: drug solution supply passage, 26: first valve, 28: sliding hole, 29: piston, 30: air valve, 31: changeover button, 40: holding device, 42: holding plate.

## Claims

1. An injection system (1) using a needleless syringe (20), comprising:
the needleless syringe (20) which injects an injection objective substance into an injection target which is a living body capable of existing in water by emitting the injection objective substance from an emission port (21) without using any injection needle;
a transfer passage (6) which is arranged in water and connects a first space which is arranged in water for accommodating a plurality of the injection targets and a second space, which is arranged in water, as a transfer destination of the plurality of injection targets, the transfer passage (6) being installed with the needleless syringe (20) so that the emission port (21) of the needleless syringe (20) is open at inside of the transfer passage (6);
a detecting unit which is capable of detecting transfer through the transfer passage (6) in order for the injection target existing in the first space to go toward the second space; and
a control unit which performs emission of the injection objective substance from the emission port (21) of the needleless syringe (20) for every injection target in accordance with the transfer of the injection target, if the transfer of the injection target is detected by the detecting unit and
a holding unit which has a holding plate (42) which extends along the transfer passage (6), and which presses the holding plate (42) against the injection target so that the injection target in the transfer passage (6) is positioned with respect to the needleless syringe (20), and which temporarily stops the transfer of the injection target in the transfer passage (6) to hold the injection target, and which allows the emission port (21) of the needless syringe (20) to abut against a body surface of the injection target on the basis of a detection result of the transfer of the injection target obtained by the detecting unit.

2. The injection system (1) using the needleless syringe (20) according to claim 1, wherein the needleless syringe (20) includes:
a syringe (24) which serves as a space for accommodating a predetermined volume of the injection objective substance;
an air cylinder which has a piston (29) formed to be capable of performing reciprocating motion by supplying and discharging pressurized air and an air valve (30) for driving the piston (29) and which pressurizes the injection objective substance accommodated in the syringe (24) by means of the reciprocating motion of the piston (29); and
a nozzle which includes the emission port (21) for emitting the injection objective substance pressurized by the piston (29) toward the injection target, wherein:
the injection system (1) further comprises a gas supply device which supplies the pressurized air for driving the piston (29) in the air cylinder to the needleless syringe (20).

3. The injection system (1) using the needleless syringe (20) according to claim 2, wherein the needleless syringe (20) includes:
a first valve (26) which allows the injection objective substance to be capable of flowing in only one direction from a vial (5) toward the syringe (24) in a communication passage formed between the syringe (24) and the vial (5) that accommodates the injection objective substance; and
a second valve (23) which allows the injection objective substance to be capable of flowing in only one direction from the syringe (24) toward the nozzle in a discharge passage (22) formed between the nozzle and the syringe (24), wherein:
the injection objective substance is supplied into the syringe (24) from the vial (5) via the communication passage when the piston (29) is moved backwardly in the air cylinder to provide a negative pressure in the syringe (24) when the first valve (26) is in an open state and the second valve (23) is in a closed state.

4. The injection system (1) using the needleless syringe (20) according to any one of claims 1 to 3, further comprising:
an obstructing unit which obstructs the injection target having transferred from the first space to the second space from returning toward the first space, the obstructing unit being provided in a predetermined passage range disposed on a side of the second space as compared with an installation portion of the emission port (21) of the needleless syringe (20) in the transfer passage (6).

5. The injection system (1) using the needleless syringe (20) according to claim 4, wherein the obstructing unit is composed of a plurality of inclined plates which are inclined toward the side of the second space as compared with the installation portion of the emission port (21) in the transfer passage (6) and which protrude to an inner side of the transfer passage (6).

6. The injection system (1) using the needleless syringe (20) according to any one of claims 1 to 5, further comprising:
an inducing device (12) which transfers the injection target existing in the first space to the second space via the transfer passage (6) .

## Patentansprüche

1. Injektionssystem (1), das eine nadellose Spritze (20) verwendet, umfassend:
die nadellose Spritze (20), die eine Injektionszielsubstanz in ein Injektionsziel injiziert, das ein lebender Körper ist, der in der Lage ist, in Wasser zu existieren, indem sie die Injektionszielsubstanz aus einer Austrittsöffnung (21) emittiert, ohne eine Injektionsnadel zu verwenden;
einen Transferdurchgang (6), der in Wasser angeordnet ist und einen ersten Raum, der in Wasser angeordnet ist, um eine Vielzahl der Injektionsziele aufzunehmen, und einen zweiten Raum, der in Wasser als ein Transferziel der Vielzahl von Injektionszielen angeordnet ist, miteinander verbindet, wobei der Transferdurchgang (6) mit der nadellosen Spritze (20) so installiert ist, dass die Austrittsöffnung (21) der nadellosen Spritze (20) an der Innenseite des Transferdurchgangs (6) offen ist;
eine Detektionseinheit, die in der Lage ist, einen Transfer durch den Transferdurchgang (6), damit das Injektionsziel, das sich in dem ersten Raum befindet, zu dem zweiten Raum gelangt, zu erfassen; und
eine Steuereinheit, die eine Emission der Injektionszielsubstanz aus der Austrittsöffnung (21) der nadellosen Spritze (20) für jedes Injektionsziel in Übereinstimmung mit dem Transfer des Injektionsziels ausführt, wenn der Transfer des Injektionsziels von der Detektionseinheit erfasst wird, und
eine Halteeinheit, die eine Halteplatte (42) aufweist, die sich entlang des Transferdurchgangs (6) erstreckt, und die die Halteplatte (42) gegen das Injektionsziel drückt, so dass das Injektionsziel in dem Transferdurchgang (6) in Bezug auf die nadellose Spritze (20) positioniert wird, und die vorübergehend den Transfer des Injektionsziels in dem Transferdurchgang (6) stoppt, um das Injektionsziel zu halten, und die es der Austrittsöffnung (21) der nadellosen Spritze (20) ermöglicht, an einer Körperoberfläche des Injektionsziels auf der Grundlage eines von der Detektionseinheit erhaltenen Erfassungsergebnisses des Transfer des Injektionsziels anzuliegen.

2. Injektionssystem (1), das die nadellose Spritze (20) verwendet, nach Anspruch 1, wobei die nadellose Spritze (20) umfasst:
eine Spritze (24), die als Raum zur Aufnahme eines vorbestimmten Volumens der Injektionszielsubstanz dient;
einen Luftzylinder mit einem Kolben (29), der so ausgebildet ist, dass er eine Hin- und Herbewegung durch Zuführen und Abgeben von Druckluft ausführen kann, und ein Luftventil (30) zum Antreiben des Kolbens (29), das die in der Spritze (24) untergebrachte Injektionszielsubstanz durch die Hin- und Herbewegung des Kolbens (29) unter Druck setzt; und
eine Düse, die die Austrittsöffnung (21) zum Ausstoßen der durch den Kolben (29) unter Druck gesetzten Injektionszielsubstanz in Richtung des Injektionsziels umfasst, wobei:
das Injektionssystem (1) weiterhin eine Gasversorgungseinrichtung umfasst, die der nadellosen Spritze (20) die Druckluft zum Antreiben des Kolbens (29) in dem Luftzylinder zuführt.

3. Injektionssystem (1), das die nadellose Spritze (20) verwendet, nach Anspruch 2, wobei die nadellose Spritze (20) umfasst:
ein erstes Ventil (26), das es ermöglicht, dass die Injektionszielsubstanz nur in einer Richtung von einer Ampulle (5) zur Spritze (24) in einem Verbindungskanal fließen kann, der zwischen der Spritze (24) und der Ampulle (5), die die Injektionszielsubstanz aufnimmt, ausgebildet ist; und
ein zweites Ventil (23), das es ermöglicht, dass die Injektionszielsubstanz in nur einer Richtung von der Spritze (24) zur Düse in einem zwischen der Düse und der Spritze (24) ausgebildeten Auslasskanal (22) fließen kann, wobei:
die Injektionszielsubstanz aus der Ampulle (5) über den Verbindungskanal in die Spritze (24) zugeführt wird, wenn der Kolben (29) in dem Luftzylinder zurückbewegt wird, um einen Unterdruck in der Spritze (24) zu erzeugen, wenn das erste Ventil (26) in einem offenen Zustand und das zweite Ventil (23) in einem geschlossenen Zustand ist.

4. Injektionssystem (1), das die nadellose Spritze (20) verwendet, nach einem der Ansprüche 1 bis 3, weiterhin umfassend:
eine Hinderniseinheit, die das Injektionsziel, das vom ersten Raum in den zweiten Raum übergegangen ist, daran hindert, zu dem ersten Raum zurückzukehren, wobei die Hinderniseinheit in einem vorbestimmten Durchgangsbereich vorgesehen ist, der im Vergleich zu einem Installationsabschnitt der Austrittsöffnung (21) der nadellosen Spritze (20) in dem Transferdurchgang (6) auf einer Seite des zweiten Raums angeordnet ist.

5. Injektionssystem (1), das die nadellose Spritze (20) verwendet, nach Anspruch 4, wobei die Hinderniseinheit aus einer Vielzahl von schrägen Platten besteht, die im Vergleich zum Einbauabschnitt der Austrittsöffnung (21) im Transferdurchgang (6) zur Seite des zweiten Raums hin geneigt sind und zu einer Innenseite des Transferdurchgangs (6) hin vorstehen.

6. Injektionssystem (1), das die nadellose Spritze (20) verwendet, nach einem der Ansprüche 1 bis 5, weiterhin umfassend:
eine induzierende Vorrichtung (12), die das in dem ersten Raum befindliche Injektionsziel über den Transferdurchgang (6) in den zweiten Raum transferiert.

## Revendications

1. Système d'injection (1) utilisant une seringue sans aiguille (20) comprenant :
- une seringue sans aiguille (20) qui injecte une substance objective d'injection dans une cible d'injection qui est un corps vivant susceptible de vivre dans l'eau en émettant la substance objective d'injection par un port d'émission (21) sans utiliser une quelconque aiguille d'injection ;
- un passage de transfert (6) organisé dans l'eau et relié à un premier espace organisé dans l'eau pour recevoir un ensemble de cibles d'injection et un second espace organisé dans l'eau comme destination de transfert de l'ensemble des cibles d'injection, le passage de transfert (6) étant équipé de la seringue sans aiguille (20) de façon que le port d'émission (21) de la seringue sans aiguille (20) s'ouvre vers l'intérieur du passage de transfert (6),
- une unité de détection pour détecter le transfert à travers le passage de transfert (6) pour la cible d'injection du premier espace passant vers le second espace, et
- une unité de commande qui effectue l'émission de la substance objective d'injection par le port d'émission (21) de la seringue sans aiguille (20) pour chaque cible d'injection en fonction du transfert de la cible d'injection lorsque le transfert de la cible d'injection est détecté par l'unité de détection, et
- une unité de maintien qui a une plaque de maintien (42) s'étendant le long du passage de transfert (6) et qui presse la plaque de maintien (42) contre la cible d'injection de façon que la cible d'injection dans le passage de transfert (6) soit positionnée par rapport à la seringue sans aiguille (20) et arrête le transfert de la cible d'injection dans le passage de transfert (6) pour tenir la cible d'injection et permet au port d'émission (21) de la seringue sans aiguille (20) de venir contre la surface du corps de la cible d'injection selon le résultat de la détection du transfert de la cible d'injection, par l'unité de détection.

2. Système d'injection (1) utilisant la seringue sans aiguille (20) selon la revendication 1,
la seringue sans aiguille (20) comprenant :
- une seringue (24) qui sert de volume pour recevoir une dose prédéterminée de la substance objective d'injection,
- un cylindre pneumatique avec un piston (29) réalisé pour effectuer un mouvement de va-et-vient en fournissant et en déchargeant l'air comprimé ainsi qu'une vanne d'air (30) pour entraîner le piston (29) et qui met en pression la substance objective d'injection dans la seringue (24) par le mouvement alternatif du piston (29), et
- une buse comportant le port d'émission (21) pour émettre la substance objective d'injection comprimée par le piston (29) vers la cible d'injection,
- le système d'injection (1) comprenant en outre un dispositif d'alimentation en gaz qui fournit l'air comprimé pour entraîner le piston (29) dans le cylindre pneumatique pour la seringue sans aiguille (20).

3. Système d'injection (1) utilisant la seringue sans aiguille (20) selon la revendication 2,
dans lequel
la seringue sans aiguille (20) comprend :
- une première valve (26) qui laisse passer la substance objective d'injection seulement dans une direction à partir d'un flacon (5) vers la seringue (24) dans un passage de communication réalisé entre la seringue (24) et le flacon (5) contenant la substance objective d'injection, et
- une seconde valve (23) qui laisse passer la substance objective d'injection seulement dans une direction à partir de la seringue (24) vers la buse dans un passage de décharge (22) réalisé entre la buse et la seringue (24), dans lequel
la substance objective d'injection est fournie à la seringue (24) à partir du flacon (5) par le passage de communication lorsque le piston (29) revient dans le cylindre pneumatique en appliquant une pression négative dans la seringue (24) lorsque la première valve (26) et en position ouverte et la seconde valve (23) est en position fermée.

4. Système de seringue (1) utilisant la seringue sans aiguille (20) selon l'une quelconque des revendications 1 à 3,
comprenant en outre :
- une unité d'obstruction qui bloque le retour vers le premier espace de la cible d'injection passée du premier espace au second espace, l'unité d'obstruction étant prévue dans une plage de passage prédéterminée sur un côté du second espace par rapport à la partie d'installation du port d'émission (21) de la seringue sans aiguille (20) dans le passage de transfert (6).

5. Système d'injection (1) utilisant la seringue sans aiguille (20) selon la revendication 4,
dans lequel
l'unité d'obstruction est composée d'un ensemble de plaques inclinées qui sont inclinées vers le côté du second espace par rapport à la partie d'installation du port d'émission (21) dans le passage de transfert (6) et qui vient en saillie à l'intérieur du passage de transfert (6).

6. Système d'injection (1) utilisant la seringue sans aiguille (20) selon l'une quelconque des revendications 1 à 5,
comprenant en outre :
- un dispositif d'induction (12) qui transfère la cible d'injection se trouvant dans le premier espace vers le second espace par le passage de transfert (6).
